# EUROPEAN PATENT SPECIFICATION

(11) **EP 4 153 298 B1**
(45) Date of publication and mention of the grant of the patent: **23.04.2025**
(21) Application number: 21724347.6
(22) Date of filing: 29.04.2021
(51) Int. Cl.: A61N 7/00, A61N 5/06, A61L 2/00

(54) **SYSTEM FOR ULTRASOUND TREATMENT COMBINED WITH UVC TEATMENT**
SYSTEM ZUR ULTRASCHALLBEHANDLUNG IN KOMBINATION MIT UV-BEHANDLUNG
SYSTÈME DE TRAITEMENT PAR ULTRASONS COMBINÉ À UN TRAITEMENT UVC

(30) Priority: 18.05.2020 FI 20205498
(43) Date of publication of application: 29.03.2023
(73) Proprietor: Innotia Eesti Oü, 10130 Tallinn (EE); Oy Pace1 Tools Ltd, 06100 Porvoo (FI)
(72) Inventor: LOKKINEN, Mika, 10130 Tallinn (EE); JOKINEN, Jukka, 06100 Porvoo (FI)
(74) Representative: Wilenius, Jami Juhani
(86) International application number: PCT/FI2021/050320
(87) International publication number: WO 2021/234214

(56) References cited:
- WO-A1-2010/101532
- CN-A- 106 377 847
- US-A1- 2014 276 247
- US-A1- 2016 074 547
- US-A1- 2018 185 529
- US-A1- 2019 184 195

## Description

### FIELD OF THE INVENTION

The invention relates to a treatment system for treating or preventing infections.

### PRIOR ART

Respiratory infections - and infections in general - are caused by viruses and bacteria which mutate over time and may eventually become resistant to antibiotics and other medications. There is a vast number of different viruses and bacteria and the type causing an infection in a patient has to be identified before a proper treatment can be determined. For some viruses and bacteria, there is no treatment available and for some the treatment is not effective enough to save all patients. Combined ultrasound and ultraviolet energy systems suitable for germicidal treatments are disclosed in documents US2006/0275171 A1, US2014/0276247 A1 and US2016/0074547 A1.

### BRIEF DESCRIPTION OF THE INVENTION

The object of the invention is a system, which can target and damage a wide variety of different viruses and bacteria and alleviate infections caused by viruses or bacteria.

Object of the invention is achieved with a two-part system where both units of the system comprise an ultrasonic transducer and at least one unit comprises ultraviolet emitter. The ultrasonic emitters are arranged to emit ultrasound waves into a pliable medium which can be pressed firmly into contact with a patient to achieve efficient propagation of ultrasound waves from the system into the body of the patient. The invention is defined in the appended set of claims.

### BRIEF DESCRIPTION OF THE FIGURES

The invention is now described in more detail in connection with preferred embodiments, with reference to the accompanying drawings, in which:
Fig. 1 shows a treatment system according to an embodiment in use;
Fig. 2 is an isometric view of a base unit according to an embodiment;
Fig. 3 shows a base unit according to an embodiment seen from above;
Fig. 4 is a section view of Section A-A shown in Figure 3;
Fig. 5 is a section view of Section B-B shown in Figure 3;
Fig. 6 is an isometric section view of a portable unit according to an embodiment;
Fig. 7 is a section view of Section C-C shown in Figure 6; and
Fig. 8 shows a portable unit according to an embodiment seen from below;

### DETAILED DESCRIPTION OF THE INVENTION

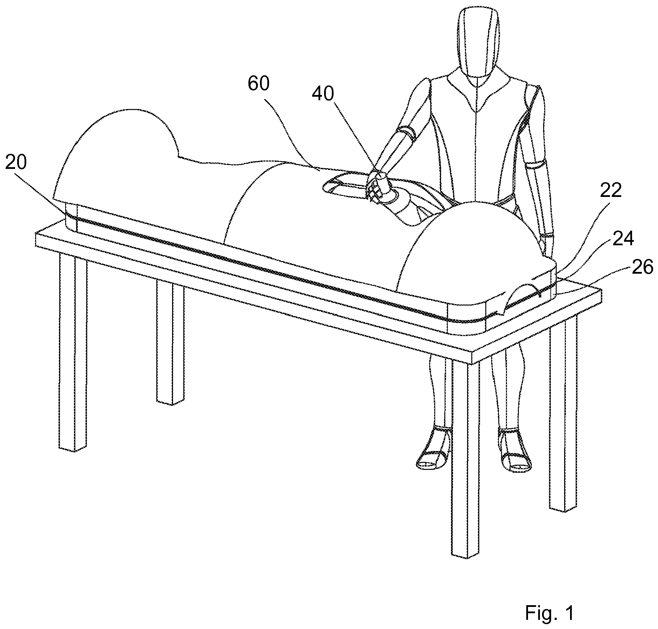

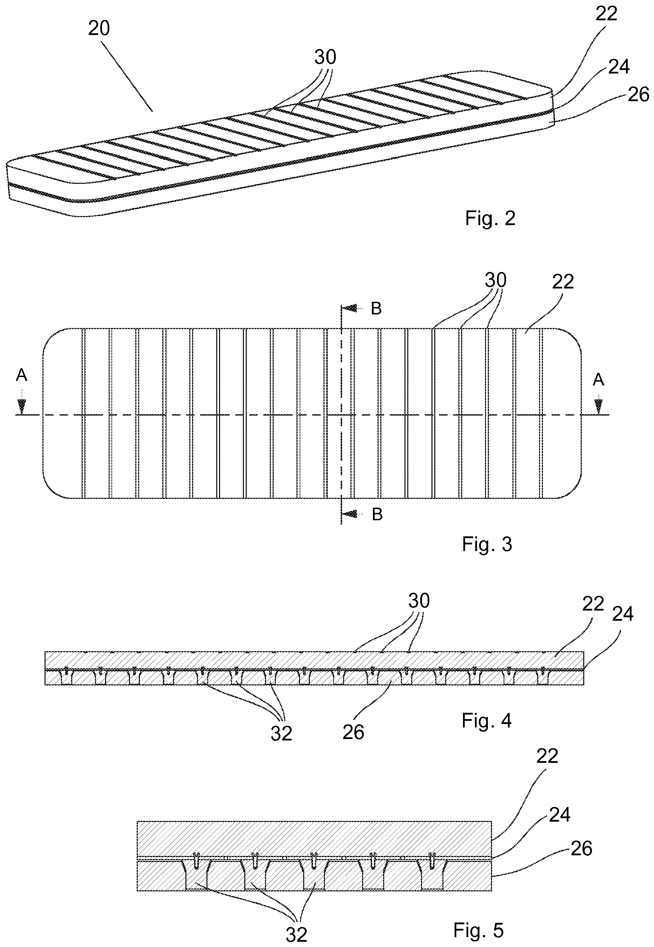

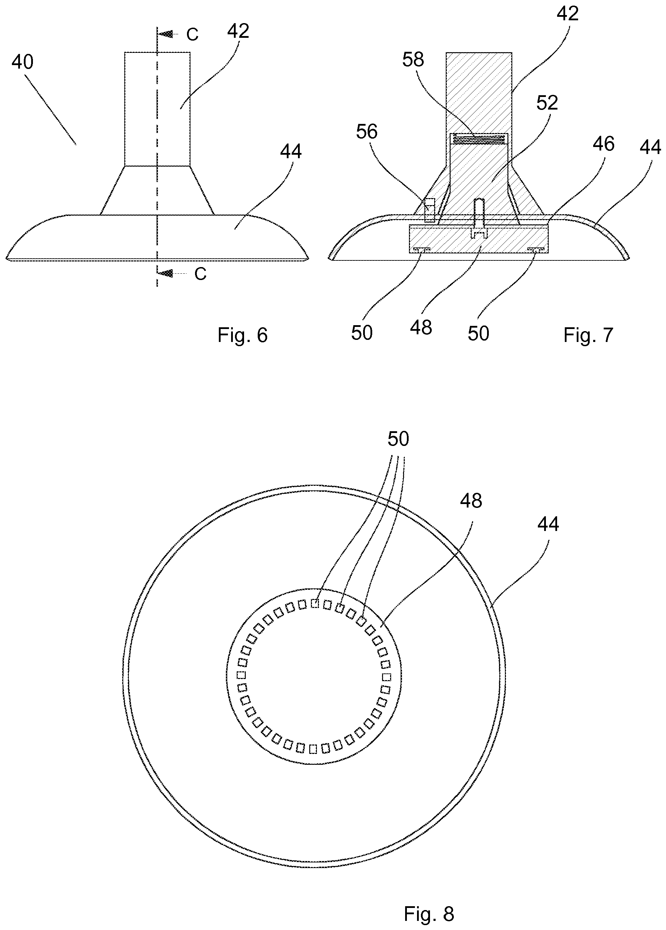

Figure 1 shows a treatment system according to an embodiment of the present disclosure. It is important to note that the treatment system is for treating a patient by causing changes within the patient's body whereas imaging devices are for creating an image of a patient's body without causing changes in the patient's body. The present disclosure is strictly related to treatment devices and systems. The treatment system comprises a base unit 20 and a portable unit 40. The details of the base unit have been shown in more detail in Figures 2-5 and the portable unit in Figures 6-8. The treatment system may also include a protective cover 60 protecting both the patient and an operator of the treatment system from unwanted ultraviolet emissions. The portable unit can be attached to the base unit but movable in relation to the base unit.

The base unit 20 (shown in Figures 2-5) can be in a form of a mattress or in an embodiment similar to the portable unit 40. The base unit comprises a pliable top layer 22 which can adapt to the shape of the patient placed against the it. The pliable top layer can be made out of silicone, silicone-based material or some other pliable material where ultrasound waves can propagate without significant losses. In an embodiment the top layer can be a plastic bladder or silicone bladder filled with water or gel.

The base unit also comprises one or more ultrasonic transducers 32 configured to produce ultrasound waves into the pliable top layer 22. The frequency of the ultrasound produced by the ultrasonic transducers can be e.g. in a range from 100 kHz to 1200 kHz. The base unit 20 also comprises a solid layer 24 which is preferably in contact with the pliable top layer 22 over a large area of the solid layer 24. The one or more ultrasonic transducers 32 are fixed to the solid layer 24. The solid layer is a metal plate, such as an aluminium plate or a steel plate which provides the base unit with rigidity and increases transmission of ultrasound waves from the ultrasonic transducers into the pliable top layer 22 which is on one side of the solid layer. On the opposite side of the solid layer 24 is a bottom layer 26. The ultrasound waves from the ultrasonic transducers 32 are prevented from entering into the bottom layer 26 by using material, such as a foam, in the bottom layer 26 where the ultrasound waves do not propagate properly. There can also be an air gap between the solid layer 24 and the bottom layer 26 to ensure that most of the ultrasonic energy is directed into the pliable top layer.

The portable unit 40 (shown in Figures 6-8) is preferably a handheld device as shown in Figure 1 and thereby it preferably comprises a handle 42. The portable unit can also be attached to a movable arm that can be manually or remotely controlled to move the portable unit in a desired direction. The portable unit 40 comprises a pliable pad 48 which can adapt to the shape of the patient placed against the it. The pliable top layer can be made out of silicone, silicone-based material or some other pliable material where ultrasound waves can propagate without significant losses. In an embodiment the top layer can be a plastic bladder or silicone bladder filled with water or gel.

The portable unit comprises an ultrasonic transducer 52 configured to produce ultrasound waves into the pliable pad 48. The frequency of the ultrasound produced by the ultrasonic transducers can be e.g. in a range from 100 kHz to 1200 kHz. The portable unit 40 preferably also comprises a solid layer 46 which is preferably in contact with the pliable pad 48 over a large area of the solid layer 46. The ultrasonic transducers 52 is preferably fixed to the solid layer 46. The solid layer can be e.g. a metal plate, such as an aluminium plate or a steel plate which increases transmission of ultrasound waves from the ultrasonic transducers into the pliable pad 48.

The treatment system further comprises a source of ionizing electromagnetic radiation 50 which may form a part of the base unit 20, part of the portable unit 40 or the source can be a separate unit from which the ionizing electromagnetic radiation is guided to the patient either directly or through the portable unit or the base unit. The source of ionizing electromagnetic radiation 50 can be, for example, an ultraviolet emitter that emits ultraviolet light, an X-ray tube that emits X-rays or a gamma ray emitter that emits gamma rays. In the following embodiments, the source of ionizing electromagnetic radiation 50 is an ultraviolet emitter but the treatment system of the present disclosure can also be used with said other types of ionizing electromagnetic radiation and the following examples are not limited to use of ultraviolet emitters only.

In an embodiment of the present disclosure, the portable unit 40 further comprises one or more ultraviolet emitters 50 as the source of ionizing electromagnetic radiation 50. Said one or more ultraviolet emitters 50 are configured to emit ultraviolet light in a wavelength range from 100 nm to 280 nm. Preferably, the ultraviolet emitters 50 are light emitting diodes (LEDs) having the highest emission peak at a wavelength range from 250 nm to 270 nm. In an embodiment, the one or more ultraviolet emitters 50 of the portable unit 40 are arranged to emit said ultraviolet light into the pliable pad 48. In this case, the pliable pad is at least partially translucent for emitted wavelengths, for example side edge(s) of the pliable pad 48 may be opaque to prevent the ultraviolet emission from escaping the pliable pad into unwanted directions. The ultraviolet emitters may also be covered with a clear plastic or glass cover for allowing the ultraviolet emission to propagate but at the same time protecting the ultraviolet emitters from direct contact with the patient and the patient from the ultraviolet emitters. The unwanted ultraviolet emission can also be blocked with a dome 44 which can be rigid or resilient dome essentially blocking any gaps between the patient and the side of the portable unit 40 that is placed against the patient.

In an embodiment, the base unit 20 also comprises one or more ultraviolet emitters 30 configured to emit ultraviolet light in a wavelength range from 100 nm to 280 nm. Preferably, also the ultraviolet emitters 30 of the base unit 20 are light emitting diodes (LEDs) having the highest emission peak at a wavelength range from 250 nm to 270 nm. Arrangements similar to the portable unit 40 can be used in the base unit when placing the ultraviolet emitters.

Since excessively large dose of ionizing electromagnetic radiation, such as ultraviolet radiation, is harmful, the dosage is preferably restricted. For example, the ultraviolet emitters 30, 50 of the system can be configured to operate at full power for example at most 100 milliseconds, 1 second or 10 seconds at a time. The restriction can also be a power restriction to certain wattage or an energy-based restriction where a patient can only receive a certain amount of energy in a single treatment.

Power levels of ultrasound and ultraviolet light can vary greatly depending on a body part that is treated with the device. For example, an infection in human fingernail can be treated with relatively low power levels compared to a respiratory infection. Even higher power levels may be needed for treating larger mammals, such as treating a horse suffering from a gastroentiritis. Therefore the power levels of the device are adjustable and should be adjusted case by case by a medical professional. Similarly, frequency of the ultrasound can be adjusted depending on a tissue that is treated to achieve desired penetration of ultrasound in to the tissue.

In an embodiment, where the base unit 20 comprises multiple ultrasonic transducers 32, the system can be configured to activate one or more of these multiple ultrasonic transducers 32 of the base unit 20 upon activation of the ultrasonic transducer 52 of the portable unit 40. The activated ultrasonic transducers can be predetermined according to a treatment plan or a set of ultrasonic transducers can be manually selected to begin transmission once the ultrasonic transducer 52 of the portable unit is activated. The activation of the portable unit can be transmitted wirelessly or by wired connection to the base unit. Alternatively, the base unit can be configured to detect the activation of the portable unit by sensing ultrasound waves with a separate detector (not shown in the drawings) or with one or more ultrasonic transducers 32 of the base unit 20.

The same principle can be applied to the ultraviolet emitters. In an embodiment, where the base unit 20 comprises multiple ultraviolet emitters 30, the system can be configured to activate one or more of the multiple ultraviolet emitters 30 of the base unit 20 upon activation of the one or more ultrasonic emitters 50 of the portable unit 40. The activated ultraviolet emitters can be predetermined according to a treatment plan or a set of ultraviolet emitters can be manually selected to begin emission once one or more of the ultraviolet emitters 50 of the portable unit are activated. The activation of the portable unit can be transmitted wirelessly or by wired connection to the base unit. Alternatively, the base unit can be configured to detect the activation of the portable unit by sensing ultraviolet emission with a separate sensor (not shown in the drawings).

In an embodiment of the present disclosure, the base unit 20 comprises multiple ultrasonic transducers 32 and said ultrasonic transducers 32 are configured to detect ultrasonic transmission of the ultrasonic transducer 52 of the portable unit 40. The treatment system is configured to selectively activate one or more of the ultrasonic transducers 32 of the base unit 20. The activated ultrasonic transducers are preferably the ones that detect the strongest signal from the portable unit 40, or alternatively the ultrasonic transducer that detects the strongest signal is activated as well as neighbouring ultrasonic transducers directly next to the one sensing the strongest signal. Selectively activating means that the number of activated ultrasonic transducers 32 is at least one but smaller than the total amount of ultrasonic transducers 32 of the base unit 20.

These embodiments of the treatment system make it possible to apply ultrasound to patient's body from opposite sides simultaneously. In an embodiment, the same applies to the ultraviolet light. This feature allows for lower power levels to be used while still penetrating the body with ultrasound or ultraviolet light. The inventors have found that the ultrasound and ultraviolet light have a strong synergistic effect on bacteria and viruses. Even the ultrasound alone has proven to be effective to reduce inflammation. The ultrasound weakens capsules of the bacteria and other outermost parts of bacteria and viruses. The weakening allows for the ultraviolet light to damage the bacteria and viruses with significantly less power compared to a case where just ultraviolet light is used. The combination of ultrasound and ultraviolet light reduces the needed light intensity by a factor of at least ten. Therefore, intensity and duration of the ultrasound and the ultraviolet light are preferably adjustable for achieving a desired result of the treatment.

The ultraviolet light, applied immediately or soon after applying the ultrasound, is intended to damage the bacteria or viruses so that reproduction of bacteria and virus replication are fully or essentially prevented. The time between the ultrasound and ultraviolet light should be less than one second and preferably the ultraviolet light is applied immediately when ultrasound is applied. Preferably the ultraviolet light is applied while the ultrasound transmission is still ongoing. In an embodiment, ultrasound is applied continuously and ultraviolet light is applied in pulses lasting less than a second or less than 100 milliseconds while ultrasound is being applied. Many of the most severe symptoms can be avoided when the bacteria cannot reproduce and viruses cannot replicate, because then the patient's immune system will be less likely to overshoot when attacking these bacteria and viruses.

The treatment system may also comprise a safety switch to protect the patient and the operator of the treatment system. The safety switch prevents accidental activation of the ultraviolet emitters of the portable unit or the whole system. In an embodiment, the pliable pad 48 and the metal plate 46 of the portable unit 40 are at rest at a first position (shown in Figure 7) where the metal plate 46 is not in contact with a pin 56. A galvanic contact between the pin 56 and the metal plate 46 is needed for activating the circuit powering the ultraviolet emitters. Thereby, in the first position the one or more ultraviolet emitters 50 are inoperable. The pliable pad 48 and the metal plate 46 are configured to be movable to a second position where the metal plate 46 is in contact with the pin 56 and thereby the one or more ultraviolet emitters 50 are operable. In an embodiment, the ultraviolet emitters 30 of the base unit 20 are also inoperable in the first position of the portable unit and operable in the second position of the portable unit. The portable unit has preferably a spring-bias towards the first position where e.g. a helical spring 58 exerts a force on the pliable pad and the metal plate which has to be overcome by exerting a higher force in the opposite direction to e.g. the pliable pad. This is typically achieved by pushing the pliable pad 48 of the portable unit against a patient.

It is obvious to the skilled person in the art that, as technology develops, the basic idea of the invention can be implemented in various ways. The invention and its embodiments are therefore not limited to only the examples presented above, rather they may vary within the scope of the claims.

## Claims

1. A treatment system comprising a base unit (20) and a portable unit (40), wherein the base unit comprises a pliable top layer (22) and one or more ultrasonic transducers (32) configured to produce ultrasound waves into the pliable top layer (22), and where the portable unit (40) comprises a pliable pad (48) and an ultrasonic transducer (52) configured to produce ultrasound waves into the pliable pad (48), wherein the treatment system further comprises a source of ionizing electromagnetic radiation (50) configured to emit ionizing electromagnetic radiation, wherein the base unit (20) further comprises a solid layer (24) and said one or more ultrasonic transducers (32) are fixed to the solid layer (24), where the solid layer (24) of the base unit (20) is a metal plate.

2. A treatment system according to claim 1, wherein the source of ionizing electromagnetic radiation is in the portable unit (40) and comprises one or more ultraviolet emitters (50) configured to emit ultraviolet light in a wavelength range from 100 nm to 280 nm.

3. A treatment system according to claim 1 or 2, wherein the source of ionizing electromagnetic radiation is in the base unit (40) and comprises one or more ultraviolet emitters (30) configured to emit ultraviolet light in a wavelength range from 100 nm to 280 nm.

4. A treatment system according to claim 2 or 3, wherein said one or more ultraviolet emitters (30, 50) are light emitting diodes having the highest emission peak at a wavelength range from 250 nm to 270 nm.

5. A treatment system according to any one of claims 2 to 4, wherein said one or more ultraviolet emitters (50) of the portable unit (40) are arranged to emit said ultraviolet light into the pliable pad (48) and the pliable pad is at least partially translucent for emitted wavelengths.

6. A treatment system according to any one of claims 1 to 5, wherein the portable unit (40) further comprises a solid layer (46) and where said ultrasonic transducer (52) is fixed to the solid layer (46).

7. A treatment system according to claim 6, wherein the solid layer (46) of the portable unit (40) is a metal plate.

8. A treatment system according to any one of claims 2 -5, wherein the ultraviolet emitters (30, 50) of the system are configured to operate at full power at most 1 second at a time.

9. A treatment system according to any one of claims 1 to 8, wherein the base unit (20) comprises multiple ultrasonic transducers (32) and the system is configured to activate one or more of the multiple ultrasonic transducers (32) of the base unit (20) upon activation of the ultrasonic transducer (52) of the portable unit (40).

10. A treatment system according to any one of claims 2 to 9, wherein the base unit (20) comprises multiple ultraviolet emitters (30) and the system is configured to activate one or more of the multiple ultraviolet emitters (30) of the base unit (20) upon activation of the one or more ultrasonic emitters (50) of the portable unit (40).

11. A treatment system according to any one of claims 1 to 10, wherein the base unit (20) comprises multiple ultrasonic transducers (32) and the ultrasonic transducers (32) of the base unit (20) are configured to detect ultrasonic transmission of the ultrasonic transducer (52) of the portable unit (40) and the treatment system is configured to selectively activate one or more of the ultrasonic transducers (32) of the base unit (20) detecting the strongest signal from the portable unit (40), where the number of activated ultrasonic transducers (32) is smaller than the total amount of ultrasonic transducers (32) of the base unit (20).

12. A treatment system according to claim 7, wherein the pliable pad (48) and the metal plate (46) are at rest at a first position where the metal plate (46) is not in contact with a pin (56) and thereby the one or more ultraviolet emitters (50) are inoperable, and the pliable pad (48) and the metal plate (46) are configured to be movable to a second position where the metal plate (46) is in contact with the pin (56) and thereby the one or more ultraviolet emitters (50) are operable.

## Patentansprüche

1. Behandlungssystem, umfassend eine Basiseinheit (20) und eine tragbare Einheit (40), wobei die Basiseinheit eine nachgiebige obere Schicht (22) und einen oder mehrere Ultraschallwandler (32), die dazu konfiguriert sind, Ultraschallwellen in die nachgiebige obere Schicht (22) zu erzeugen, umfasst, und wobei die tragbare Einheit (40) eine nachgiebige Kontaktfläche (48) und einen Ultraschallwandler (52), der dazu konfiguriert ist, Ultraschallwellen in die nachgiebige Kontaktfläche (48) zu erzeugen, umfasst, wobei das Behandlungssystem ferner eine Quelle von ionisierender elektromagnetischer Strahlung (50) umfasst, die dazu konfiguriert ist, eine ionisierende elektromagnetische Strahlung zu emittieren, wobei die Basiseinheit (20) ferner eine feste Schicht (24) umfasst, und der eine oder die mehreren Ultraschallwandler (32) an der festen Schicht (24) befestigt sind, wobei die feste Schicht (24) der Basiseinheit (20) eine Metallplatte ist.

2. Behandlungssystem nach Anspruch 1, wobei sich die Quelle von ionisierender elektromagnetischer Strahlung in der tragbaren Einheit (40) befindet und einen oder mehrere ultraviolette Emitter (50) umfasst, die dazu konfiguriert sind, ultraviolettes Licht in einem Wellenlängenbereich von 100 nm bis 280 nm zu emittieren.

3. Behandlungssystem nach Anspruch 1 oder 2, wobei sich die Quelle von ionisierender elektromagnetischer Strahlung in der Basiseinheit (40) befindet und einen oder mehrere ultraviolette Emitter (30) umfasst, die dazu konfiguriert sind, ultraviolettes Licht in einem Wellenlängenbereich von 100 nm bis 280 nm zu emittieren.

4. Behandlungssystem nach Anspruch 2 oder 3, wobei der eine oder die mehreren ultravioletten Emitter (30, 50) Leuchtdioden sind, welche die höchste Emissionsspitze in einem Wellenlängenbereich von 250 nm bis 270 nm aufweisen.

5. Behandlungssystem nach einem der Ansprüche 2 bis 4, wobei der eine oder die mehreren ultravioletten Emitter (50) der tragbaren Einheit (40) dazu angeordnet sind, das ultraviolette Licht in die nachgiebige Kontaktfläche (48) zu emittieren, und die nachgiebige Kontaktfläche für die emittierten Wellenlängen mindestens teilweise durchlässig ist.

6. Behandlungssystem nach einem der Ansprüche 1 bis 5, wobei die tragbare Einheit (40) ferner eine feste Schicht (46) umfasst, und wobei der Ultraschallwandler (52) an der festen Schicht (46) befestigt ist.

7. Behandlungssystem nach Anspruch 6, wobei die feste Schicht (46) der tragbaren Einheit (40) eine Metallplate ist.

8. Behandlungssystem nach einem der Ansprüche 2 bis 5, wobei die ultravioletten Emitter (30, 50) des Systems dazu konfiguriert sind, mit voller Leistung für höchstens 1 Sekunde auf einmal zu funktionieren.

9. Behandlungssystem nach einem der Ansprüche 1 bis 8, wobei die Basiseinheit (20) mehrere Ultraschallwandler (32) umfasst, und das System dazu konfiguriert ist, einen oder mehrere der mehreren Ultraschallwandler (32) der Basiseinheit (20) bei Aktivierung des Ultraschallwandlers (52) der tragbaren Einheit (40) zu aktivieren.

10. Behandlungssystem nach einem der Ansprüche 2 bis 9, wobei die Basiseinheit (20) mehrere ultraviolette Emitter (30) umfasst, und das System dazu konfiguriert ist, einen oder mehrere der mehreren ultravioletten Emitter (30) der Basiseinheit (20) bei Aktivierung des einen oder der mehreren Ultraschallemitter (50) der tragbaren Einheit (40) zu aktivieren.

11. Behandlungssystem nach einem der Ansprüche 1 bis 10, wobei die Basiseinheit (20) mehrere Ultraschallwandler (32) umfasst, und die Ultraschallwandler (32) der Basiseinheit (20) dazu konfiguriert sind, eine Ultraschallübertragung des Ultraschallwandlers (52) der tragbaren Einheit (40) zu detektieren, und das Behandlungssystem dazu konfiguriert ist, selektiv einen oder mehrere der Ultraschallwandler (32) der Basiseinheit (20) zu aktivieren, die das stärkste Signal aus der tragbaren Einheit (40) detektiert, wobei die Anzahl von aktivierten Ultraschallwandlern (32) kleiner als die Gesamtmenge der Ultraschallwandler (32) der Basiseinheit (20) ist.

12. Behandlungssystem nach Anspruch 7, wobei sich die nachgiebige Kontaktfläche (48) und die Metallplatte (46) in Ruhestellung befinden in einer ersten Position, in der die Metallplatte (46) nicht mit einem Stift (56) in Kontakt steht, und dadurch der eine oder die mehreren ultravioletten Emitter (46) nicht betriebsfähig sind, und die nachgiebige Kontaktfläche (58) und die Metallplatte (46) dazu konfiguriert sind, in eine zweite Position bewegbar zu sein, in der die Metallplatte (46) mit dem Stift (56) in Kontakt steht, und dadurch der eine oder die mehreren ultravioletten Emitter (50) betriebsfähig sind.

## Revendications

1. Système de traitement comprenant une unité de base (20) et une unité portable (40), dans lequel l'unité de base comprend une couche supérieure souple (22) et un ou plusieurs transducteurs à ultrasons (32) configurés pour produire des ondes ultrasonores dans la couche supérieure souple (22), et dans lequel l'unité portable (40) comprend un coussinet souple (48) et un transducteur à ultrasons (52) configuré pour produire des ondes ultrasonores dans le coussinet souple (48), dans lequel le système de traitement comprend en outre une source de rayonnement électromagnétique ionisant (50) configurée pour émettre un rayonnement électromagnétique ionisant, dans lequel l'unité de base (20) comprend en outre une couche solide (24) et les un ou plusieurs transducteurs à ultrasons (32) sont fixés à la couche solide (24), la couche solide (24) de l'unité de base (20) étant une plaque métallique.

2. Système de traitement selon la revendication 1, dans lequel la source de rayonnement électromagnétique ionisant se trouve dans l'unité portable (40) et comprend un ou plusieurs émetteurs d'ultraviolets (50) configurés pour émettre de la lumière ultraviolette dans une plage de longueurs d'onde de 100 nm à 280 nm.

3. Système de traitement selon la revendication 1 ou 2, dans lequel la source de rayonnement électromagnétique ionisant se trouve dans l'unité de base (40) et comprend un ou plusieurs émetteurs d'ultraviolets (30) configurés pour émettre de la lumière ultraviolette dans une plage de longueurs d'onde de 100 nm à 280 nm.

4. Système de traitement selon la revendication 2 ou 3, dans lequel les un ou plusieurs émetteurs d'ultraviolets (30, 50) sont des diodes électroluminescentes ayant le pic d'émission le plus élevé dans une plage de longueurs d'onde de 250 nm à 270 nm.

5. Système de traitement selon l'une quelconque des revendications 2 à 4, dans lequel les un ou plusieurs émetteurs d'ultraviolets (50) de l'unité portable (40) sont agencés pour émettre ladite lumière ultraviolette dans le coussinet souple (48) et le coussinet souple est au moins partiellement translucide aux longueurs d'onde émises.

6. Système de traitement selon l'une quelconque des revendications 1 à 5, dans lequel l'unité portable (40) comprend en outre une couche solide (46), et dans lequel ledit transducteur à ultrasons (52) est fixé à la couche solide (46).

7. Système de traitement selon la revendication 6, dans lequel la couche solide (46) de l'unité portable (40) est une plaque métallique.

8. Système de traitement selon l'une quelconque des revendications 2 à 5, dans lequel les émetteurs d'ultraviolets (30, 50) du système sont configurés pour fonctionner à pleine puissance pendant au plus 1 seconde à la fois.

9. Système de traitement selon l'une quelconque des revendications 1 à 8, dans lequel l'unité de base (20) comprend plusieurs transducteurs à ultrasons (32) et le système est configuré pour activer un ou plusieurs des plusieurs transducteurs à ultrasons (32) de l'unité de base (20) lors de l'activation du transducteur à ultrasons (52) de l'unité portable (40).

10. Système de traitement selon l'une quelconque des revendications 2 à 9, dans lequel l'unité de base (20) comprend plusieurs émetteurs d'ultraviolets (30) et le système est configuré pour activer un ou plusieurs des plusieurs émetteurs d'ultraviolets (30) de l'unité de base (20) lors de l'activation des un ou plusieurs émetteurs ultrasonores (50) de l'unité portable (40).

11. Système de traitement selon l'une quelconque des revendications 1 à 10, dans lequel l'unité de base (20) comprend plusieurs transducteurs à ultrasons (32) et les transducteurs à ultrason (32) de l'unité de base (20) sont configurés pour détecter une transmission ultrasonore du transducteur à ultrasons (52) de l'unité portable (40), et le système de traitement est configuré pour activer sélectivement un ou plusieurs des transducteurs à ultrasons (32) de l'unité de base (20) qui détecte le signal le plus fort provenant de l'unité portable (40), le nombre de transducteurs à ultrasons (32) activés étant inférieur à la quantité totale des transducteurs à ultrasons (32) de l'unité de base (20).

12. Système de traitement selon la revendication 7, dans lequel le coussinet souple (48) et la plaque métallique (46) sont au repos dans une première position dans laquelle la plaque métallique (46) n'est pas en contact avec une broche (56), et de ce fait les un ou plusieurs émetteurs d'ultraviolets (50) ne sont pas opérationnels, et le coussinet souple (48) et la plaque métallique (46) sont configurés pour pouvoir être déplacés dans une deuxième position dans laquelle la plaque métallique (46) est en contact avec la broche (56), et de ce fait les un ou plusieurs émetteurs d'ultraviolets (50) sont opérationnels.
